(19) **Europäisches Patentamt**

**European Patent Office**

**Office européen des brevets**

(11) **EP 0 861 819 B1**

(12) **FASCICULE DE BREVET EUROPEEN**

(45) Date de publication et mention
de la délivrance du brevet:
**20.09.2000 Bulletin 2000/38**

(51) Int Cl.⁷: **C07C 45/28**, C07C 47/22

(21) Numéro de dépôt: **98400313.7**

(22) Date de dépôt: **11.02.1998**

(54) **Procédé de fabrication d'acroléine à partir du propylène par réaction redox**

Verfahren zur Herstellung von Acrolein aus Propylen durch Redox Reaktion

Process for the preparation of acrolein from propylene by redox reaction

(84) Etats contractants désignés:
**AT BE CH DE DK ES FI FR GB GR IE IT LI LU NL PT SE**

(30) Priorité: **27.02.1997 FR 9702343**

(43) Date de publication de la demande:
**02.09.1998 Bulletin 1998/36**

(73) Titulaire: **Atofina**
**92800 Puteaux (FR)**

(72) Inventeurs:
• **Hecquet, Gérard**
**62400 Bethune (FR)**
• **Schirmann, Jean-Pierre**
**75011 Paris (FR)**
• **Simon, Michel**
**57500 Saint-Avold (FR)**
• **Descat, Gilles**
**57500 Saint-Avold (FR)**
• **Etienne, Eric**
**57500 Saint-Avold (FR)**

(74) Mandataire: **Rieux, Michel et al**
**Atofina**
**D.C.R.D./D.P.I.**
**4, Cours Michelet,**
**La Défense 10**
**92091 Paris la Défense Cedex (FR)**

(56) Documents cités:
EP-A- 0 169 449    EP-A- 0 450 596
FR-A- 2 725 715    US-A- 4 341 717

# EP 0 861 819 B1

**Description**

La présente invention concerne la fabrication de l'acroléine à partir du propylène par oxydation selon une réaction redox. L'invention concerne également l'utilisation d'une composition solide d'oxydes mixtes comme système redox dans ladite réaction.

La production industrielle de l'acroléine est à l'heure actuelle effectuée par oxydation catalytique du propylène en phase vapeur. Tous les efforts de perfectionnement de ce procédé ont porté jusqu'ici sur la mise au point de catalyseurs donnant un taux de conversion le plus élevé possible du propylène ainsi qu'une sélectivité la plus élevée possible pour l'acroléine désirée.

Ainsi, le brevet français n° 2 093 773 décrit la fabrication d'acroléine par oxydation catalytique du propylène en phase vapeur, avec de l'oxygène moléculaire, en présence d'un oxyde catalytique dont la composition des éléments catalytiques, exprimée en rapport atomique, est la suivante :

$$Co_{2,0-20,0}Fe_{0,1-10,0}Bi_{0,1-10,0}W_{0,5-10,0}Mo_{2,0-11,5}Si_{0,5-15,0}Z_{0,005-1,0}$$

avec W + Mo = 12,0 et Z représentant un métal alcalin. On peut préparer ce catalyseur en mélangeant des solutions aqueuses de molybdate d'ammonium et de paratungstate d'ammonium, en ajoutant au mélange aqueux des solutions de nitrate de cobalt, de nitrate de fer et de nitrate de bismuth, puis en ajoutant une solution aqueuse d'hydroxyde ou de carbonate d'un métal alcalin, puis de la silice colloïdale comme source de silicium, en moulant la substance obtenue et en la calcinant à 350-600°C dans un courant d'air.

Le brevet américain n° 3 855 308 décrit la préparation de l'acroléine par oxydation catalytique en phase vapeur du propylène avec l'oxygène moléculaire, en présence d'un oxyde catalytique dont la composition des éléments catalytiques, exprimée en rapport atomique, est la suivante :

$$Co_{2,0-20,0}Fe_{0,1-10,0}Bi_{0,1-10,0}W_{0,5-10,0}Mo_{2,0-11,5}Si_{0,5-15,0}Tl_{0,005-3,0}Z_{0-3,0}$$

W + Mo étant égal à 12,0 et Z représentant un métal alcalin ou alcalino-terreux.

Les matières premières utilisées pour former le catalyseur peuvent être les oxydes des différents métaux, mais aussi, selon le cas, les nitrates, carbonates, hydroxydes. Dans le cas de Mo et W, les sels d'acides tels que le molybdate d'ammonium et le tungstate d'ammonium sont préconisés. Ainsi un catalyseur selon ce brevet américain est préparé en mélangeant des solutions aqueuses respectivement de molybdate d'ammonium et de paratungstate d'ammonium, en ajoutant des solutions respectivement de nitrate de cobalt, de nitrate de fer et de nitrate de bismuth, puis une solution aqueuse d'hydroxyde ou de carbonate de métal alcalin, puis de la silice colloïdale comme source de silicium, en concentrant le système par évaporation, en ajoutant un support si nécessaire, et en faisant suivre par une évaporation, un mélange de la substance résultante et une calcination à 350-600°C.

Le brevet japonais Showa 45-125 359 décrit un procédé de fabrication d'acroléine en phase vapeur par oxydation catalytique du propylène par l'air ou l'oxygène, en présence d'un catalyseur de formule :

$$Ni_aCo_bFe_cBi_dMe_eH_hMo_fO_g$$

dans laquelle :

- a = 0 - 20 ; b = 0 - 20 avec a + b compris entre 0,5 et 20 ; c = 0,5 - 8 ; d = 0,1 - 7 ; 0 < e ≤ 2 ; h = 0 - 0,3 ; f = 12 ; g = 36 - 90 ;
- Me est l'un parmi Sn, Zn, W, Cr, Mn et Ti ; et
- H est au moins l'un parmi K, Rb et Cs.

Pour préparer ce catalyseur, on peut ajouter à une solution aqueuse d'un composé du molybdène (molybdate d'ammonium, acide molybdique ou oxyde de molybdène), des solutions aqueuses de composés de Ni, Co, Fe, K (et/ou Rb, Cs), Bi et Me, puis ajouter un support tel que l'alumine, le carbure de silicium et la silice (sol de silice ou gel de silice), puis on chauffe le mélange résultant à siccité, on le calcine à environ 500°C, et on le transforme en pastilles.

La Société déposante a maintenant découvert que l'on peut fabriquer l'acroléine par oxydation du propylène en phase gazeuse en l'absence d'oxygène moléculaire, en faisant passer le propylène sur une composition solide d'oxydes mixtes particulière, laquelle agit comme système redox et fournit l'oxygène nécessaire à la réaction.

Les avantages de ce nouveau procédé sont les suivants :

- la limitation de la suroxydation des produits formés qui a lieu en présence d'oxygène moléculaire ; selon la présente invention, du fait que l'on opère en l'absence d'oxygène moléculaire, la formation de $CO_x$ (monoxyde de carbone et dioxyde de carbone), produits de dégradation, est réduite, ce qui permet d'augmenter la sélectivité en acroléine, comme montré ci-après par les Exemples Comparatifs 4, 8, 12 et 16 ;
- la sélectivité en acroléine reste bonne lorsque le taux de réduction de la composition solide augmente ;
- la composition solide, une fois qu'elle a subi une réduction et une perte progressive de son activité, est facilement régénérable par chauffage en présence d'oxygène ou d'un gaz contenant de l'oxygène après une certaine période d'utilisation ; après la régénération, le solide retrouve son activité initiale et peut être utilisé dans un nouveau cycle de réaction ;
- la séparation des étapes de réduction de la composition solide et de régénération de celle-ci permet :

  - d'augmenter la sélectivité en acroléine ; et
  - d'augmenter la pression partielle en propylène, une telle pression partielle d'alimentation en propylène n'étant plus limitée par l'existence d'une zone explosive par le mélange propylène + oxygène.

La présente invention a donc d'abord pour objet l'utilisation d'une composition solide d'oxydes mixtes de formule (I) :

$$Mo_{12}W_aBi_bFe_cCo_dNi_eSi_fK_gSn_hO_x \qquad \text{(I)}$$

dans laquelle :

- a est compris entre 0 et 5, bornes incluses ;
- b est compris entre 0,5 et 5, bornes incluses ;
- c est compris entre 0,1 et 10, bornes incluses ;
- d est compris entre 0,5 et 10, bornes incluses ;
- e est compris entre 0 et 10, bornes incluses ;
- f est compris entre 0 et 15, bornes incluses ;
- g est compris entre 0 et 1, bornes incluses ;
- h est compris entre 0 et 2, bornes incluses ; et
- x est la quantité d'oxygène lié aux autres éléments et dépend de leurs états d'oxydation,

dans la fabrication de l'acroléine par oxydation du propylène, ladite composition solide réagissant avec le propylène selon la réaction redox (1) :

$$SOLIDE_{oxydé} + Propylène \rightarrow SOLIDE_{réduit} + Acroléine \qquad \text{(1).}$$

Les oxydes des différents métaux entrant dans la composition de l'oxyde mixte de formule (I) peuvent être utilisés comme matières premières dans la préparation de cette composition, mais les matières premières ne sont pas limitées aux oxydes ; comme autres matières premières, on peut citer :

- dans le cas du molybdène, le molybdate d'ammonium et l'acide molybdique, et dans le cas du tungstène, le tungstate d'ammonium et l'acide tungstique ;
- dans le cas du cobalt, du bismuth, du nickel et du fer, les nitrates, carbonates et hydroxydes, tels que le nitrate de cobalt, le nitrate de bismuth, le nitrate de nickel, le nitrate ferrique ;
- dans le cas de l'étain, le chlorure d'étain et l'hydroxyde d'étain ; et
- dans le cas du potassium, l'hydroxyde, le carbonate ou le nitrate de potassium ;

et, d'une manière générale, tous les composés susceptibles de former un oxyde par calcination, à savoir sels métalliques d'acides organiques, sels métalliques d'acides minéraux, composés métalliques complexes, composés métalliques organiques, etc.

La source de silicium est généralement constituée par de la silice colloïdale.

La présente invention a pour objet un procédé de fabrication de l'acroléine à partir du propylène, procédé suivant lequel on fait passer du propylène gazeux sur une composition solide de formule (I) telle que définie ci-dessus, pour conduire la réaction redox (1) telle qu'indiquée ci-dessus, en opérant à une température de 200 à 600°C, notamment de 250 à 450°C, sous une pression de $1,01 \times 10^4$ à $1,01 \times 10^6$ Pa (0,1 à 10 atmosphères), notamment de $5,05 \times 10^4$ à $5,05 \times 10^5$ Pa (0,5 - 5 atmosphères) et avec un temps de séjour de 0,01 seconde à 90 secondes, notamment de 0,1 seconde à 30 secondes, en l'absence d'oxygène moléculaire.

Conformément à des modes de réalisation particuliers de la présente invention, on peut introduire le propylène gazeux en mélange avec un gaz inerte, tel que l'azote, et/ou avec de l'eau (vapeur d'eau).

Au cours de la réaction redox (1), la composition solide subit une réduction et une perte progressive de son activité. C'est pourquoi, une fois que la composition solide est passée à l'état réduit, on conduit la régénération de ladite composition solide selon la réaction (2) :

$$\text{SOLIDE}_{\text{réduit}} + O_2 \rightarrow \text{SOLIDE}_{\text{oxydé}} \tag{2}$$

par chauffage en présence d'un excès d'oxygène ou d'un gaz contenant de l'oxygène à une température de 250 à 500°C, pendant le temps nécessaire à la réoxydation de la composition solide.

Après la régénération, qui peut être effectuée dans des conditions de température et de pression identiques à ou différentes de celles de la réaction redox, la composition solide retrouve une activité initiale et peut être utilisée dans un nouveau cycle de réaction.

On peut conduire la réaction redox (1) et la régénération dans un dispositif à deux étages, à savoir un réacteur et un régénérateur qui fonctionnent simultanément et dans lesquels alternent périodiquement deux charges de composition solide ; on peut également conduire la réaction redox (1) et la régénération dans un même réacteur en alternant les périodes de réaction et de régénération.

La préparation de l'acroléine selon l'invention s'effectue selon une réaction stoechiométrique et non catalytique.

Les Exemples suivants illustrent la présente invention sans toutefois en limiter la portée. Dans les formules indiquées dans ces Exemples, x est la quantité d'oxygène lié aux autres éléments et dépend de leurs états d'oxydation.

Les conversions, sélectivités et rendements sont définis comme suit :

$$\text{Conversion (\%)} = \frac{\text{Nombre de moles de propylène ayant réagi}}{\text{Nombre de moles de propylène introduites}} \times 100$$

$$\text{Sélectivité (\%) en acroléine} = \frac{\text{Nombre de moles d'acroléine formées}}{\text{Nombre de moles de propylène ayant réagi}} \times 100$$

$$\text{Sélectivité (\%) en acide acrylique} = \frac{\text{Nombre de moles d'acide acrylique formées}}{\text{Nombre de moles de propylène ayant réagi}} \times 100$$

### EXEMPLE 1

(a) <u>Préparation d'un solide de formule</u>

$$Mo_{12}Co_{4,7}Bi_1Ni_{2,6}Fe_{3,7}W_{0,5}Sn_{0,5}Si_1K_{0,08}O_x$$

A température ambiante, on dissout 618 g de $Co(NO_3)_2.6H_2O$, 343 g de $Ni(NO_3)_2.6H_2O$, 674 g de $Fe(NO_3)_3.9H_2O$ et 3,8 g de $KNO_3$ dans 1250 ml d'eau distillée. Egalement à température ambiante, on dissout 230 g de $Bi(NO_3)_3.5H_2O$ dans 300 ml d'eau distillée acidifiée avec 50 ml de $HNO_3$ à 68% en volume. Toujours à température ambiante, on dissout 53 g de $SnCl_2.2H_2O$ dans 60 ml d'eau distillée. A 40°C, on dissout 956,2 g de $(NH_4)_6Mo_7O_{24}.4H_2O$ dans 2600 ml d'eau distillée.

On verse successivement, sous agitation, la solution contenant le bismuth et celle contenant l'étain dans la solution contenant Co, Ni, Fe et K. La solution résultante est ensuite versée, toujours sous agitation, dans la solution contenant le molybdène. On y ajoute alors en pluie 71 g de silice colloïdale (à 40% en masse) et 55,6 g de $WO_3$. On chauffe le mélange résultant à 90°C pendant 1,5 heure, puis on le sèche à 140°C pendant 12 heures. Le solide obtenu est calciné 6 heures à 500°C sous air. Les différents métaux sont présents dans ce solide dans les rapports atomiques tels qu'indiqués dans l'intitulé de cet exemple.

(b) Préparation d'acroléine à partir du propylène par réaction redox

Dans un réacteur tubulaire à 400°C, on charge 200 mg de ce solide, puis on le balaye par un flux continu de 12 ml/min d'hélium. On injecte 2,3 x $10^{-6}$ mole de propylène sur le solide. Le propylène est converti à 91,5%, avec des sélectivités en acroléine et acide acrylique de respectivement 80,0% et 4,0%.

## EXEMPLE 2

Après avoir conduit la réaction de l'Exemple 1(b), on soumet à nouveau le même solide à quatre injections successives de propylène, dans les mêmes conditions de test que dans l'Exemple 1. Les performances obtenues sont rapportées dans le Tableau 1.

Tableau 1

| N° d'injection | Conversion du propylène (%) | Sélectivité en acroléine (%) | Sélectivité en acide acrylique (%) |
|---|---|---|---|
| 1 | 89,4 | 81,0 | 4,0 |
| 2 | 87,3 | 79,1 | 3,8 |
| 3 | 85,5 | 80,1 | 3,8 |
| 4 | 83,7 | 78,9 | 3,7 |

## EXEMPLE 3

Après le traitement réducteur de l'Exemple 2, le solide est régénéré 1 heure sous air à 400°C, puis replacé sous flux d'hélium. Quatre nouvelles injections successives de 2,3 x $10^{-6}$ mole de propylène sont envoyées sur le solide. Les performances obtenues sont rapportées dans le Tableau 2.

Tableau 2

| N° d'injection | Conversion de propylène (%) | Sélectivité en acroléine (%) | Sélectivité en acide acrylique (%) |
|---|---|---|---|
| 1 | 93,3 | 80,7 | 4,4 |
| 2 | 90,6 | 81,0 | 4,2 |
| 3 | 88,2 | 80,4 | 4,0 |
| 4 | 86,0 | 80,0 | 3,9 |

## EXEMPLE 4 (comparatif)

On charge 200 mg d'un solide préparé selon l'Exemple 1 dans un réacteur tubulaire à 400°C, puis on le balaye par un flux continu de 12 ml/min. d'air. On injecte 2,3 x $10^{-6}$ mole de propylène sur le solide. Le propylène est converti à 92,9 % avec des sélectivités en acroléine et acide acrylique de respectivement 70,4% et 3,4%.

## EXEMPLE 5

(a) Préparation d'un solide de formule

$$Mo_{12}Co_{3,5}Bi_{1,1}Fe_{0,8}W_{0,5}Si_{1,4}K_{0,05}O_x$$

On dissout 60,9 g de $Co(NO_3)_2.6H_2O$ dans 20 ml d'eau distillée.
On dissout également 20,2 g de $Fe(NO_3)_3.9H_2O$ dans 15 ml d'eau distillée, et 31,2 g de $Bi(NO_3)_3.5H_2O$ dans 30 ml d'eau distillée acidifiée par 6 ml de $HNO_3$ à 68% en volume.
Séparément, on dissout en chauffant et en agitant 127,4 g de $(NH_4)_6Mo_7O_{24}.4H_2O$ dans 150 ml d'eau, puis on ajoute 7,4 g de $WO_3$.
On introduit goutte à goutte en 20 minutes la solution aqueuse contenant le cobalt dans la solution aqueuse des sels d'ammonium. On introduit ensuite la solution ferrique en 10 minutes, puis la solution contenant le bismuth en 15 minutes. Dans le gel résultant, on ajoute, en 10 minutes, une solution obtenue en dissolvant 0,2 g de KOH et 12,8 g de silice colloïdale (à 40% en masse) dans 15 ml d'eau. On malaxe le gel ainsi obtenu pendant 1 heure à température ambiante, puis 1 heure à 70°C.

Le gel est ensuite séché à 130°C pendant 18 heures. Le solide obtenu est calciné 9 heures à 450°C sous air. Les différents métaux sont présents dans ce solide dans les rapports atomiques tels qu'indiqués dans l'intitulé de l'exemple.

(b) Préparation d'acroléine à partir du propylène par réaction redox

Dans un réacteur tubulaire à 400°C, on charge 200 mg de ce solide, puis on le balaye par un flux continu de 12 ml/min d'hélium. On injecte $2,3 \times 10^{-6}$ mole de propylène sur le solide. Le propylène est converti à 82,8% avec des sélectivités en acroléine et acide acrylique de respectivement 77,7% et 5,1%.

EXEMPLE 6

Après avoir conduit la réaction de l'Exemple 5(b), on soumet à nouveau le même solide à quatre injections successives de propylène, dans les mêmes conditions de test que l'Exemple 5. Les performances obtenues sont rapportées dans le Tableau 3.

Tableau 3

| N° d'injection | Conversion du propylène (%) | Sélectivité en acroléine (%) | Sélectivité en acide acrylique (%) |
|---|---|---|---|
| 1 | 79,8 | 77,8 | 5,1 |
| 2 | 75,4 | 76,5 | 4,8 |
| 3 | 72,0 | 75,7 | 4,5 |
| 4 | 69,3 | 75,4 | 4,6 |

EXEMPLE 7

Après le traitement réducteur de l'Exemple 6, le solide est régénéré 1 heure sous air à 400°C puis replacé sous flux d'hélium. Quatre nouvelles injections de $2,3 \times 10^{-6}$ mole de propylène sont envoyées sur le solide. Les performances obtenues sont rapportées dans le Tableau 4.

Tableau 4

| N° d'injection | Conversion du propylène (%) | Sélectivité en acroléine (%) | Sélectivité en acide acrylique (%) |
|---|---|---|---|
| 1 | 83,9 | 78,5 | 5,6 |
| 2 | 77,7 | 77,9 | 5,2 |
| 3 | 72,6 | 76,2 | 4,6 |
| 4 | 69,5 | 74,7 | 4,4 |

EXEMPLE 8 (Comparatif)

On charge 200 mg d'un solide préparé selon l'Exemple 5 dans un réacteur tubulaire à 400°C, puis on le balaye par un flux continu de 12 ml/min, d'air. On injecte $2,3 \times 10^{-6}$ mole de propylène sur le solide. Le propylène est converti à 84,9% avec des sélectivités en acroléine et acide acrylique de respectivement 66,1% et 5,0%.

EXEMPLE 9

(a) Préparation d'un solide de formule

$$Mo_{12}Co_{3,5}Bi_{1,1}Fe_{0,8}W_{0,5}Si_{1,4}K_{0,05}O_x$$

On prépare le solide ci-dessus de la même manière que dans l'Exemple 5, mais en remplaçant les 7,4 g de WO$_3$ par 8,1 g de paratungstate d'ammonium.

(b) Préparation d'acroléine à partir du propylène par réaction redox

On utilise ce solide pour la réaction de l'Exemple 5 avec les résultats suivants : le propylène est converti à 92,1%, avec des sélectivités en acroléine et acide acrylique de respectivement 72,7% et 8,0%.

EXEMPLE 10

Après avoir conduit la réaction de l'Exemple 9(b), on soumet à nouveau le même solide à quatre injections successives de propylène, dans les mêmes conditions de test que dans l'Exemple 9. Les performances obtenues sont rapportées dans le Tableau 5.

Tableau 5

| N° d'injection | Conversion du propylène (%) | Sélectivité en acroléine (%) | Sélectivité en acide acrylique (%) |
|---|---|---|---|
| 1 | 89,8 | 72,4 | 7,4 |
| 2 | 86,4 | 73,6 | 7,0 |
| 3 | 83,7 | 71,2 | 6,7 |
| 4 | 80,5 | 70,7 | 6,3 |

EXEMPLE 11

Après le traitement réducteur de l'Exemple 10, le solide est régénéré 1 heure sous air à 400°C, puis replacé sous flux d'hélium. Quatre nouvelles injections successives de $2,3 \times 10^{-6}$ mole de propylène sont envoyées sur le solide. Les performances obtenues sont rapportées dans le Tableau 6.

Tableau 6

| N° d'injection | Conversion du propylène (%) | Sélectivité en acroléine (%) | Sélectivité en acide acrylique (%) |
|---|---|---|---|
| 1 | 90,7 | 73,4 | 7,8 |
| 2 | 87,2 | 74,3 | 7,1 |
| 3 | 83,3 | 72,4 | 6,5 |
| 4 | 80,3 | 70,7 | 6,2 |

EXEMPLE 12 (comparatif)

On charge 200 mg d'un solide préparé selon l'Exemple 9 dans un réacteur tubulaire à 400°C, puis on le balaye par un flux continu de 12 ml/min d'air. On injecte $2,3 \times 10^{-6}$ mole de propylène sur le solide. Le propylène est converti à 91,3% avec des sélectivités en acroléine et acide acrylique de respectivement 61,0% et 6,9%.

EXEMPLE 13

(a) Préparation d'un solide de formule

$$Mo_{12}Co_{3,8}Bi_{1,2}Fe_{0,9}W_{1,1}Si_{1,5}K_{0,05}O_x$$

On prépare le solide ci-dessus de la même manière que dans l'Exemple 5.

(b) Préparation d'acroléine à partir du propylène par réaction redox

On utilise ce solide pour la réaction de l'Exemple 5 avec les résultats suivants : le propylène est converti à 90,4%, avec des sélectivités en acroléine et acide acrylique de respectivement 78,1% et 6,3%.

EXEMPLE 14

Après avoir conduit la réaction de l'Exemple 13(b), on soumet à nouveau le même solide à quatre injections successives de propylène, dans les mêmes conditions de test que dans l'Exemple 13. Les performances obtenues sont rapportées dans le Tableau 7.

Tableau 7

| N° d'injection | Conversion du propylène (%) | Sélectivité en acroléine (%) | Sélectivité en acide acrylique (%) |
|---|---|---|---|
| 1 | 85,0 | 78,7 | 5,3 |
| 2 | 80,9 | 78,0 | 4,9 |
| 3 | 76,8 | 77,5 | 4,8 |
| 4 | 73,4 | 76,9 | 4,5 |

EXEMPLE 15

Après le traitement réducteur de l'Exemple 14, le solide est régénéré 1 heure sous air à 400°C, puis replacé sous flux d'hélium. Quatre nouvelles injections de $2,3 \times 10^{-6}$ mole de propylène sont envoyées sur le solide. Les performances obtenues sont rapportées dans le Tableau 8.

Tableau 8

| N° d'injection | Conversion du propylène (%) | Sélectivité en acroléine (%) | Sélectivité en acide acrylique (%) |
|---|---|---|---|
| 1 | 91,3 | 78,1 | 7,7 |
| 2 | 88,7 | 79,6 | 6,3 |
| 3 | 84,6 | 78,2 | 5,3 |
| 4 | 80,2 | 78,3 | 4,8 |

EXEMPLE 16 (comparatif)

On charge 200 mg d'un solide préparé selon l'Exemple 9 dans un réacteur tubulaire à 400°C, puis on le balaye par un flux continu de 12 ml/min. d'air. On injecte $2,3 \times 10^{-6}$ mole de propylène sur le solide. Le propylène est converti à 91,8%, avec des sélectivités en acroléine et acide acrylique de respectivement 71,0% et 6,6%.

**Revendications**

**1.** Utilisation d'une composition solide d'oxydes mixtes de formule (I) :

$$Mo_{12}W_aBi_bFe_cCo_dNi_eSi_fK_gSn_hO_x \qquad (I)$$

dans laquelle :

- a est compris entre 0 et 5, bornes incluses ;
- b est compris entre 0,5 et 5, bornes incluses ;
- c est compris entre 0,1 et 10, bornes incluses ;
- d est compris entre 0,5 et 10, bornes incluses ;
- e est compris entre 0 et 10, bornes incluses ;
- f est compris entre 0 et 15, bornes incluses ;
- g est compris entre 0 et 1, bornes incluses ;
- h est compris entre 0 et 2, bornes incluses ; et
- x est la quantité d'oxygène lié aux autres éléments et dépend de leurs états d'oxydation,

dans la fabrication de l'acroléine par oxydation du propylène, ladite composition solide réagissant avec le propylène selon la réaction redox (1) :

$$\text{SOLIDE}_{oxydé} + \text{Propylène} \rightarrow \text{SOLIDE}_{réduit} + \text{Acroléine} \qquad (1).$$

2. Procédé de fabrication de l'acroléine à partir du propylène, caractérisé par le fait que l'on fait passer du propylène gazeux sur une composition solide de formule (I) telle que définie à la revendication 1, pour conduire la réaction redox (1) telle qu'indiquée à la revendication 1, en opérant à une température de 200 à 600°C, sous une pression de $1,01 \times 10^4$ à $1,01 \times 10^6$ Pa (0,1 à 10 atmosphères), et avec un temps de séjour de 0,01 seconde à 90 secondes, en l'absence d'oxygène moléculaire.

3. Procédé selon la revendication 2, caractérisé par le fait que l'on introduit le propylène gazeux en mélange avec un gaz inerte tel que l'azote et/ou avec de l'eau.

4. Procédé selon l'une des revendications 2 et 3, caractérisé par le fait que l'on conduit la réaction redox (1) à une température de 250 à 450°C.

5. Procédé selon l'une des revendications 2 à 4, caractérisé par le fait que l'on conduit la réaction redox (1) sous une pression de $5,05 \times 10^4$ - $5,05 \times 10^5$ Pa (0,5 - 5 atmosphères).

6. Procédé selon l'une des revendications 2 à 5, caractérisé par le fait que l'on conduit la réaction redox (1) avec un temps de séjour de 0,1 seconde à 30 secondes.

7. Procédé selon l'une des revendications 2 à 6, caractérisé par le fait qu'une fois que la composition solide est passée à l'état réduit, on conduit la régénération de ladite composition solide selon la réaction (2) :

$$\text{SOLIDE}_{réduit} + O_2 \rightarrow \text{SOLIDE}_{oxydé} \qquad (2)$$

par chauffage en présence d'un excès d'oxygène ou d'un gaz contenant de l'oxygène à une température de 250 à 500°C, pendant le temps nécessaire à la réoxydation de la composition solide.

8. Procédé selon la revendication 7, caractérisé par le fait que l'on conduit la réaction redox (1) et la régénération dans un dispositif à deux étages, à savoir un réacteur et un régénérateur qui fonctionnent simultanément et dans lesquels alternent périodiquement deux charges de composition solide.

9. Procédé selon la revendication 7, caractérisé par le fait que l'on conduit la réaction redox (1) et la régénération dans un même réacteur en alternant les périodes de réaction et de régénération.

**EP 0 861 819 B1**

**Claims**

1.  Use of a solid mixed oxide composition of formula (I):

$$Mo_{12}W_aBi_bFe_cCo_dNi_eSi_fK_gSn_hO_x \qquad (I)$$

in which:

- a is between 0 and 5, limits included,
- b is between 0.5 and 5, limits included,
- c is between 0.1 and 10, limits included,
- d is between 0.5 and 10, limits included,
- e is between 0 and 10, limits included,
- f is between 0 and 15, limits included,
- g is between 0 and 1, limits included,
- h is between 0 and 2, limits included, and
- x is the quantity of oxygen bonded to the other elements and depends on their oxidation states,

in the manufacture of acrolein by propylene oxidation, the said solid composition reacting with the propylene according to the redox reaction (1):

$$solid_{oxidized} + propylene \rightarrow solid_{reduced} + acrolein \qquad (1).$$

2.  Process for the manufacture of acrolein from propylene, characterized in that gaseous propylene is passed over a solid composition of formula (I) as defined in Claim 1, to conduct the redox reaction (1) as indicated in Claim 1, while operating at a temperature of 200 to 600°C, at a pressure of $1.01 \times 10^4$ to $1.01 \times 10^6$ Pa (0.1 to 10 atmospheres) and with a residence time of 0.01 second to 90 seconds, in the absence of molecular oxygen.

3.  Process according to Claim 2, characterized in that the gaseous propylene is introduced as a mixture with an inert gas such as nitrogen and/or with water.

4.  Process according to either of Claims 2 and 3, characterized in that the redox reaction (1) is conducted at a temperature of 250 to 450°C.

5.  Process according to one of Claims 2 to 4, characterized in that the redox reaction (1) is conducted at a pressure of $5.05 \times 10^4$ - $5.05 \times 10^5$ Pa (0.5-5 atmospheres).

6.  Process according to one of Claims 2 to 5, characterized in that the redox reaction (1) is conducted with a residence time of 0.1 second to 30 seconds.

7.  Process according to one of Claims 2 to 6, characterized in that, once the solid composition has changed to the reduced state, the regeneration of the said solid composition is conducted according to reaction (2):

$$solid_{reduced} + O_2 \rightarrow solid_{oxidized} \qquad (2)$$

by heating in the presence of an excess of oxygen or of an oxygen-containing gas at a temperature of 250 to 500°C, for the time necessary for the reoxidation of the solid composition.

8.  Process according to Claim 7, characterized in that the redox reaction (1) and the regeneration are conducted in a two-stage device, namely a reactor and a regenerator which function simultaneously and in which two charges of solid composition alternate periodically.

9.  Process according to Claim 7, characterized in that the redox reaction (1) and the regeneration are conducted in the same reactor by alternating the reaction and regeneration periods.

**Patentansprüche**

1.  Verwendung einer festen Zusammensetzung gemischter Oxide der Formel (I)

$$Mo_{12}W_aBi_bFe_cCo_dNi_eSi_fK_gSn_hO_x \tag{I},$$

in der

-   a im Bereich von 0 bis 5 einschließlich der Grenzwerte,
-   b im Bereich von 0,5 bis 5 einschließlich der Grenzwerte,
-   c im Bereich von 0,1 bis 10 einschließlich der Grenzwerte,
-   d im Bereich von 0,5 bis 10 einschließlich der Grenzwerte,
-   e im Bereich von 0 bis 10 einschließlich der Grenzwerte
-   f im Bereich von 0 bis 15 einschließlich der Grenzwerte,
-   g im Bereich von 0 bis 1 einschließlich der Grenzwerte,
-   h im Bereich von 0 bis 2 einschließlich der Grenzwerte liegt und
-   x die Menge an Sauerstoff ist, die mit den anderen Elementen verbunden ist und von ihrem Oxidationszustand abhängt,

    zur Herstellung von Acrolein durch Oxidation von Propylen, wobei die feste Zusammensetzung mit dem Propylen gemäß der Redox-Reaktion (1)

$$Feststoff_{oxidiert} + Propylen \rightarrow Feststoff_{reduziert} + Acrolein \tag{1}$$

    reagiert.

2.  Verfahren zur Herstellung von Acrolein aus Propylen, dadurch gekennzeichnet, daß man gasförmiges Propylen über/durch die wie in Anspruch 1 definierte feste Zusammensetzung der Formel (I) strömen läßt, um die wie in Anspruch 1 angegebene Redox-Reaktion (1) durchzuführen, wobei die Reaktion bei einer Temperatur von 200 bis 600 °C und einem Druck von $1,01 \cdot 10^4$ bis $1,01 \cdot 10^6$ Pa (0,1 bis 10 atm) und mit einer Verweilzeit von 0,01 bis 90 s in Abwesenheit von molekularem Sauerstoff durchgeführt wird.

3.  Verfahren nach Anspruch 2, dadurch gekennzeichnet, daß das gasförmige Propylen im Gemisch mit einem inerten Gas, wie Stickstoff, und/oder mit Wasser zugeführt wird.

4.  Verfahren nach einem der Ansprüche 2 und 3, dadurch gekennzeichnet, daß die Redox-Reaktion (1) bei einer Temperatur von 250 bis 450 °C durchgeführt wird.

5.  Verfahren nach einem der Ansprüche 2 bis 4, dadurch gekennzeichnet, daß die Redox-Reaktion (1) bei einem Druck von $5,05 \cdot 10^4$ bis $5,05 \cdot 10^5$ Pa (0,5 bis 5 atm) durchgeführt wird.

6.  Verfahren nach einem der Ansprüche 2 bis 5, dadurch gekennzeichnet, daß die Redox-Reaktion (1) mit einer Verweilzeit von 0,1 bis 30 s durchgeführt wird.

7.  Verfahren nach einem der Ansprüche 2 bis 6, dadurch gekennzeichnet, daß nach Überführung der festen Zusammensetzung in den reduzierten Zustand die Regeneration der festen Zusammensetzung gemäß der Reaktion (2)

$$Feststoff_{reduziert} + O_2 \rightarrow Feststoff_{oxidiert} \tag{2}$$

    durch Erhitzen in Gegenwart eines Überschusses an Sauerstoff oder eines Gases, das Sauerstoff enthält, auf eine Temperatur von 250 bis 500 °C während eines Zeitraums, der für die Oxidation der festen Zusammensetzung erforderlich ist, durchgeführt wird.

8.  Verfahren nach Anspruch 7, dadurch gekennzeichnet, daß die Redox-Reaktion (1) und die Regenerierung in einer Vorrichtung mit zwei Bereichen, nämlich einem Reaktor und einem Regenerator, durchgeführt werden, die gleichzeitig in Betrieb sind und zwischen denen zwei Chargen der festen Zusammensetzung periodisch wechseln.

9. Verfahren nach Anspruch 7, dadurch gekennzeichnet, daß die Redox-Reaktion (1) und die Regenerierung in einem einzigen Reaktor durchgeführt werden, wobei die Zeitintervalle, in denen die Reaktion bzw. die Regeneration stattfindet, einander abwechseln.